(19) **Europäisches Patentamt / European Patent Office / Office européen des brevets**

(11) **EP 3 434 182 B1**

(12) **EUROPEAN PATENT SPECIFICATION**

(45) Date of publication and mention
of the grant of the patent:
**11.09.2019 Bulletin 2019/37**

(51) Int Cl.:
**A61B 5/026** (2006.01)     **A61B 5/00** (2006.01)

(21) Application number: **18183024.1**

(22) Date of filing: **11.07.2018**

(54) **SYSTEM FOR MONITORING THE BLOOD SUPPLY TO THE TRANSPLANTED ORGAN**

**SYSTEM ZUR ÜBERWACHUNG DER BLUTVERSORGUNG DES TRANSPLANTIERTEN ORGANS**

**SYSTEME DE SURVEILLANCE DE L'APPROVISIONNEMENT EN SANG DE L'ORGANE TRANSPLANTE**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.07.2017 CZ 20170430**

(43) Date of publication of application:
**30.01.2019 Bulletin 2019/05**

(73) Proprietor: **Ceské vysoké ucení technické v Praze 16000 Praha 6- Dejvice (CZ)**

(72) Inventors:
• **BORTEL, Radoslav**
  **96001 Zvolen (SK)**
• **HOSPODKA, Jiri**
  **27341 Brandysek (CZ)**
• **SEBEK, Jan**
  **16000 Praha 6 (CZ)**
• **FRONEK, Jiri**
  **15500 Praha 5 - Reporyje (CZ)**
• **JANOUSEK, Libor**
  **14800 Praha 4 - Kunratice (CZ)**
• **MALY, Stepan**
  **10900 Praha 10 - Petrovice (CZ)**

(74) Representative: **Kratochvil, Vaclav
Patent and Trademark Office
P.O. Box 26
295 01 Mnichovo Hradiste (CZ)**

(56) References cited:
**WO-A2-2015/070162     US-A- 4 653 498
US-A1- 2015 208 923**

**Description**

Background of the Invention

The present solution deals with systems for monitoring changes in the function of internal organs by minimally invasive methods. For example, it is a task of monitoring a renal graft perfusion in an early postoperative period using an optical method inside a living organism, especially a human body.

Description of Prior Art

At present, ultrasound examination is used in clinical practice to monitor the state of organs during postoperative hospitalization. Besides the need to provide personnel and the fact that this examination offers only one-off measurement without the capability of automated continuous checking, its main disadvantage is that the accuracy of the determination of the target organ condition depends to a large extent on the experience of the operator and his subjective evaluation.

For the purposes of ongoing monitoring of the state of organs, experimental electronic pulse oximetry systems can be used to measure oxygen saturation in internal organs. This topic is discussed in the following studies: Theodor, Michael, et al. "Implantable pulse oximetry on subcutaneous tissue." Engineering in Medicine and Biology Society (EMBC), 2014 36th Annual International Conference of the IEEE. IEEE, 2014 and Kyriacou, P. A., M. Hickey, and J. P. Phillips. "Pulse oximetry of body cavities and organs." Engineering in Medicine and Biology Society (EMBC), 2013 35th Annual International Conference of the IEEE. IEEE, 2013. These systems are based on monitoring the amplitude of quasi-periodic changes in the reflectance or transmittance of the monitored tissue caused by the varying concentrations of oxyhemoglobin and deoxyhemoglobin during a heart cycle. The ratio of the magnitude of these changes on at least two suitably chosen wavelengths, typically 650nm and 900nm, is then proportional to the value of the blood oxygenation. This measurement method has several drawbacks. Changes in the reflectance/transmittance intensity during the heart cycle are relatively small and can easily disappear in the interference caused by the instability of the optical link between the sensor and the measured tissue, which is often caused by even small and virtually unavoidable mechanical motion of the sensor. Another disadvantage is the unreliability of the measurement at a low perfusion value, see papers Shah, Nitin, et al. "Performance of three new-generation pulse oximeters during motion and low perfusion in volunteers." Journal of clinical anesthesia 24.5 (2012): 385-391 a Barker, Steven J. ""Motion-resistant" pulse oximetry: a comparison of new and old models." Anesthesia & Analgesia 95.4 (2002): 967-972. In this case, the pulse amplitude is lower and its measurement is unreliable, which may subsequently result in an evaluation of the incorrect oxygen saturation value, which does not indicate a problem with reduced perfusion. Because of these shortcomings, pulse oximetry is not perfectly suitable for monitoring organ perfusion.

Another principle applicable to perfusion monitoring is the measurement of the photoplethysmographic PPG signal, as described for instance in Reichelt, Stephan, et al. "Development of an implantable pulse oximeter." IEEE transactions on Biomedical Engineering 55.2 (2008): 581-588. The principle of this measurement is closely related to pulse oximetry - the monitoring of changes in transmissivity of the monitored tissue during the heart cycle is used. However, the measured signal does not indicate the oxygen saturation rate, but only the mentioned volumetric changes of the monitored organ or tissue. The disadvantage of this approach is, as with pulse oximetry, considerable sensitivity to the mechanical instability of the measuring system. Another difficulty is the possibility of false pulse detection even with reduced perfusion of the monitored organ - e.g. when a renal vein becomes blocked, the pulse of the PPG signal can still be measured both on the renal artery and on the kidney as such because the renal artery and the organ are still affected by change in blood pressure during the heart cycle. Therefore, also the PPG measurement is not a trouble-free approach to monitoring blood organ perfusion.

Another possible approach is to monitor the mechanical properties of the monitored tissue and to derive the perfusion from these mechanical properties. This approach is used in US2015208923A1, where the evaluation of mechanical properties is based on the pulse waveform analysis in the measured signal. This approach has a similar disadvantage as the pulse oximetry systems, where the pulsations can disappear due to the interference that is caused by the mechanical movement of the sensor. Moreover, the device described in US2015208923A1 does not contain an output indicating the tissue perfusion, and the information about the perfusion would have to be derived from the measured mechanical properties, which is not an optimal approach.

A common problem with some systems using measuring methods similar to oximetry and PPG is also that they use only a limited number of wavelengths. For example, WO2015070162A2 and US2009156918A1 use only two wavelengths (US2009156918A1 admits the use of also the third wavelength). Such a limited number of wavelengths does not allow for sufficiently robust monitoring of spectral characteristics, especially when measurements at individual wavelengths are affected by mechanical movement and vibration. In this case, the robustness of the system can be increased by using multiple wavelengths, at which reflectance and transmittance are measured.

Another problem with some existing devices is that they only work in the red and infrared spectrum range. For instance, the device described in WO2015070162A2 is limited to wavelengths of 650-1000nm. Better results can be achieved if wavelengths in the remaining visible spectrum are also used. These wavelengths may not reflect the change in the concentration of oxygenated and deoxygenated hemoglobin but here the reference measurements can be made, which the final classifier will use for suppression of the effects of mechanical motion and vibrations of the measuring sensor.

Although pulse oximetry and PPG have a number of drawbacks, the principle of measuring the monitored tissue by means of reflectance and transmittance at various wavelengths does include the information about the perfusion of the monitored organ. The techniques for measuring these parameters are therefore still relevant and their overview is given in the following paragraphs.

A typical arrangement of the reflectance/transmittance measurement used in both pulse oximetry systems and PPG systems always consists of irradiating the organ by transmitting circuit of the system that typically has several sources of radiation, such as LEDs, at different wavelengths, in combination with synchronized reception of the reflected/transmitted radiation from the tissue by a photosensitive sensor at the side of the receiving circuitry block.

For simultaneous measurement at several wavelengths, multiple approaches may be used. Most often, it is a time division multiplex (TDM), where the sources at individual wavelengths are activated sequentially for a predetermined elementary measuring interval, during which the measurement is performed only at a single wavelength, as described for instance in patents US4653498, US4167331, US4883353, US5792052, EP2442709, EP0329196, EP1237467, EP2022394. The result of the measurement is the mean intensity of the detected radiation during the elementary measuring interval. The disadvantage of this approach is that it can be easily affected by external interference such as power-line interference, low-frequency noise of electronics, which is typically a so-called 1/f noise, and a dark current of sensors. Another measurement approach is the frequency division multiplex (FDM), where the sources emit radiation continuously, but their power is amplitude-modulated at different frequencies. This approach is used for example in patents US8150487, US8718737, US9351673, US5482036, US5490505, US5431159, US7062307, EP1709902. After measurement, the signals corresponding to the individual wavelengths are separated by linear filtering. The advantage of this approach is the possibility of avoiding frequency bands with interference, but considerable drawbacks are the high requirements for linearity of the radiation sources and the measuring sensor. If a light source or measuring sensor is non-linear, such as a light-emitting diode or a photodiode, it is no longer possible to separate the individual components by linear filtering, especially if the measurements are made at a greater number of wavelengths. Another, less frequent alternative to the measurement is the phase multiplex described in patent US5349952, wherein the sources emit radiation continuously, while their output is amplitude-modulated by harmonic signals at one frequency with a mutual phase shift, which makes them orthogonal. After measurement, the signals are subsequently separated by a synchronous detector. The disadvantage of this approach is the possibility of using only two wavelengths because the mutual orthogonality on one frequency can only be achieved for one phase shift, namely 90°.

From a mechanical point of view, the implantable systems described for instance in article Theodor, Michael, et al. "Implantable pulse oximetry on subcutaneous tissue."

Engineering in Medicine and Biology Society (EMBC), 2014 36th Annual International Conference of the IEEE. IEEE, 2014, or in article Kyriacou, P. A., M. Hickey, and J. P. Phillips. "Pulse oximetry of body cavities and organs." Engineering in Medicine and Biology Society (EMBC), 2013 35th Annual International Conference of the IEEE. IEEE, 2013, typically feature the setback that they measure relatively small volume of the monitored tissue. Measurement systems typically consist of one sensor that measures tissue reflectance/transmittance especially in its immediate vicinity, and the measurement can therefore be affected by a mere local deterioration of the blood perfusion, which however does not reflect the condition of the entire organ.

Overall, none of the currently available solutions for monitoring the blood perfusion of the transplanted organ is without problems. Systems detecting the tissue reflectance/transmittance are primarily designed to measure pulse oximetry or PPG, which however may not reliably indicate the rate of blood perfusion. Reflectance/transmittance measurement techniques as such also have a number of drawbacks, such as noise sensitivity, or high linearity requirements for the sensor used. Last but not least, the mechanical design of the sensor limits the measurement to a small volume of the monitored tissue, which complicates the diagnostics of the organ as a whole.

## Summary of the Invention

The above-mentioned disadvantages are removed by the system for monitoring the blood supply to a transplanted organ according to the presented solution. The system consists of three sections. The transmitting section with transmitters with light-emitting diodes, which irradiate the monitored tissue by radiation of multiple wavelengths, the receiving section with receivers with photosensitive sensors, which detect the radiation reflected from the monitored tissue, and the control section, which generates the excitation pulses for the transmitters and processes signals from the receivers. The main principle of the new solution lies in the following arrangement. Each of the transmitters, which are $N$ in total where $N$ corresponds to the number of wavelengths used for irradiation, comprises at its input a digital-to-analog converter connected to the input of the voltage/current converter, the output of which is connected to at least one light-emitting diode for the wavelength of the given transmitter. If multiple light-emitting diodes are used, they are connected in series between the output of the voltage/current converter and the power supply voltage. Each of the receivers in the receiving section, which are $M$ in total where $M$ is given by the requirement for the volume of the monitored organ or its part, is formed by the photosensitive sensor, the output of which is connected via the current/voltage converter and via band-pass filter to the input of an analog-to-digital converter. The output of the analog-to-digital converter is the output of the given receiver. Each of the outputs of the receivers is connected to the first input of its corresponding demodulator in the control section. The demodulator comprises at its input a multiplier connected via the integrator with resetting and via the sampling switch to the input of its switcher. Each switcher has $N$ outputs and to each of them a feature estimator for each wavelength is connected. Each feature estimator is formed by two parallel branches. The first branch is formed at its input by a high-pass filter, the output of which is connected via the squarer circuit to the input of the first low-pass filter, the output of which is the output of the signal corresponding to the amplitude of pulsations of the radiation reflected at the given wavelength. The second branch is formed at its input by the second low-pass filter and its output is the output of the signal corresponding to slow changes of the reflected radiation intensity. The outputs of all feature estimators are connected to the classifier. The control section further contains a digital filter, while its transfer function corresponds to discretized transfer function of the analog band-pass filter. The output of the digital filter is connected to the second inputs of the demodulators, and to its input is connected the output of the generator, which is simultaneously connected to the input of the common switcher, which has outputs for the connection to the inputs of individual transmitters, so that they can be sequentially activated during the elementary measuring interval $T_p$ by the pulse $u[n]$ with the sampling frequency $f_s$ according to the equation

$$u[n] = w[n] \cdot (\sin(\Omega_0 n) + k),$$

where $w[n]$ is the weight window with length $N_w$ samples, $\Omega_0$ is the central normalized frequency of the pulse, and $k > 1$ is a constant, whereas for the total length of the pulse $u[n]$ holds that $T_p \cdot f_s > N_w$.

In a preferred embodiment, the mechanical design of the unit with sensors of the system that is applied on the monitored organ is implemented in the form of a flexible strip. This strip carries the light-emitting diodes and photosensitive sensors. The light-emitting diodes are arranged into $L$ groups where each group includes one source for each wavelength. Photosensitive sensors are placed between the individual groups. The higher the number of groups of light-emitting diodes and photosensitive sensors, the larger the volume of the monitored organ, and the whole unit is encapsulated in an optically transparent package.

The proposed system is based on monitoring the tissue reflectance or transmittance, while it includes three innovations that allow for more accurate measurement and more reliable diagnostics of the monitored organ.

The first innovation is the change in the monitored quantities on the basis of which the perfusion in the tissue is evaluated. Instead of the amplitude of the pulsation during the heart cycle, i.e. the quantities measured by the pulse oximetry and the photoplethysmographic PPG signal, the slow and long-term changes of reflectance/transmittance at several wavelengths in the visible and infrared spectra are primarily monitored. While these quantities reflect neither the immediate level of the blood oxygenation nor the tissue volume changes, they reflect changes in the spectral properties of the monitored organ caused by the change in its perfusion. In addition, these changes are even greater by orders of magnitude than changes caused by varying oxy/deoxyhemoglobin concentrations during the heart cycle, and their measurement is therefore less affected by the mechanical instability of the measuring sensors.

As a complementary measurement, the presented device can also measure changes in amplitude pulsation during the heart cycle. However, these are considered as supplementary information only and do not carry the main information which the device functions are based on.

The second innovation is a time multiplex technique with improved modulation of power transmitted between the transmitter and the receiver. This modulation minimizes the effect of added noise, sensitivity to so-called 1/f noise, allowing for robust detection of received pulses of light and infrared radiation. Due to a greater signal-to-noise ratio, this detection method features an increased sensitivity to reflected radiation from the tissue and allows to increase the distance between the transmitters and the receivers, which further increases the irradiated surface of the organ and allows the whole measurement to be carried out in a larger volume of the tissue being monitored.

The third innovation is the concept of the mechanical design of the unit with sensors of the device, allowing for easy measurement at multiple areas of the organ simultaneously, which significantly increases the volume of tissue being monitored and makes the entire monitoring robust to local changes in the organ structure. The mechanical design of the unit with sensors takes the form of a flexible strip and it is proposed in such a way that it can be removed from the patient's body without surgical intervention after the measurement is finished.

Explanation of Drawings

The system for monitoring the blood supply to a transplanted organ according to the presented solution will be further described by means of drawings. Fig. 1 shows a block diagram of the system for continuous monitoring of perfusion to a target organ. Fig. 2 shows an internal block diagram of the transmitter block. Fig. 3 shows an internal block diagram of the receiver block. Fig. 4 shows an internal block diagram of the demodulator block and Fig. 5 shows an internal block connection of the estimator block. Fig. 6 shows an example of the circuitry implementation of the transmitter and Fig. 7 shows an example of the circuitry implementation of the receiver. Fig. 8 illustrates the excitation signal $u(t)$ for the light-emitting diodes, while Fig. 9 depicts the arrangement of the implemented mechanical design of the unit with sensors of the device.

Detailed Description of the Preferred Embodiments

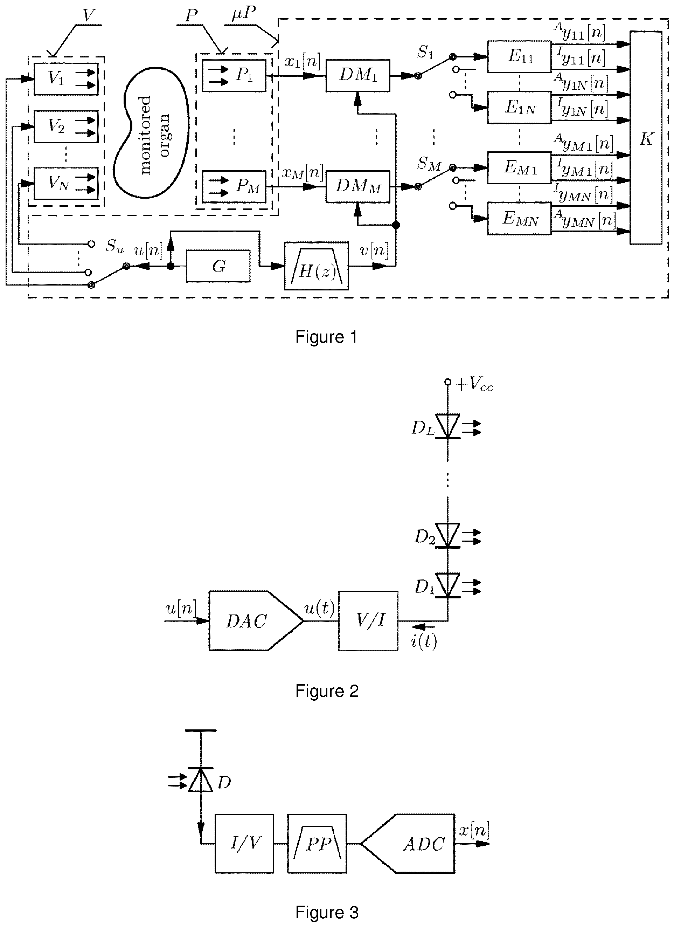

Block diagram of the system is shown in Fig. 1. The system consists of the transmitting section $\underline{V}$ with transmitters $V_1$ to $V_N$ with light-emitting diodes $D_1$ to $D_L$ used for the irradiation of the monitored tissue by radiation at various wavelengths, of the receiver section $\overline{P}$ with receivers $P_1$ to $P_M$ with photosensitive sensor $D$ used for the detection of the radiation reflected from the monitored tissue, and of the control section $\mu P$ used for generating the excitation pulses $u[n]$ for the transmitters $V_1$ to $V_N$ and for processing the signals from the receivers $P_1$ to $P_M$. Each of the transmitters $V_1$ to $V_N$, see Fig. 2, which are $N$ in total where $N$ corresponds to the number of wavelengths used for irradiation, comprises at its input a digital-to-analog converter $\underline{DAC}$ connected to the input of the voltage/current converter $\underline{V/I}$. The output of this voltage/current converter $\underline{V/I}$ is connected to at least one light-emitting diode $D_1$ to $D_L$ for the wavelength of the given transmitter $V_1$ to $V_N$. If multiple light-emitting diodes $D_1$ to $D_L$ are used, they are connected in series between the output of the voltage/current converter $\underline{V/I}$ and the power supply voltage $+ V_{cc}$. Each of the receivers $P_1$ to $P_M$ in the receiving section $\underline{P}$, see Fig. 3, which are $M$ in total, where $M$ is given by the requirement for the volume of the monitored organ or its part, is formed by the photosensitive sensor $\underline{D}$, the output of which is connected via the current/voltage converter $\underline{I/V}$ and via the analog band-pass filter $\underline{PP}$ to the input of the analog-to-digital converter $\underline{ADC}$. The output of the analog-to-digital converter $\underline{ADC}$ is the output of the corresponding receiver. Each of the outputs of receivers $P_1$ to $P_M$ is connected to the first input of their corresponding demodulators $DM_1$ to $DM_M$ in the control section $\mu P$. Individual demodulators comprise at their inputs the multiplier $\underline{MP}$ connected via the integrator $\underline{I}$ with resetting and via the sampling switch $\underline{S}$ to the input of their relevant switchers $S_1$ to $S_M$, each of which has $N$ outputs each of which is connected to one feature estimator $E_{i1}$ to $E_{iN}$ for each wavelength. Each feature estimator $E_{i1}$ to $E_{iN}$ is formed by two parallel branches. The first branch is formed at its input by the high-pass filter $\underline{HP}$, the output of which is connected via the squarer circuit $\underline{KV}$ to the input of the first low-pass filter $DP_1$. The output of the first low-pass filter $DP_1$ is the output of the signal corresponding to the amplitude of pulsations of the radiation reflected at the given wavelength. The second branch is formed at its input by the second low-pass filter $DP_2$ and its output is the output of the signal corresponding to slow changes of the reflected radiation intensity. The outputs of all feature estimators $E_{i1}$ to $E_{iN}$ are connected to the classifier $\underline{K}$. The control section $\mu P$ further contains a digital filter $\underline{H(z)}$, while its transfer function corresponds to the discretized transfer function of the analog band-pass filter $\underline{PP}$. The output of the digital filter $\underline{H(z)}$ is connected to the second inputs of the demodulators $DM_1$ to $DM_M$, and its input is connected to the output of the generator $\underline{G}$. This output is also connected to the input of the common switcher $S_u$, which has outputs for connection to the inputs of individual transmitters $V_1$ to $V_N$, so that they can be sequentially activated during the elementary measuring interval $T_p$ by the pulse $u[n]$ with the sampling frequency $f_s$. The generator $\underline{G}$ synthesizes the signal according to the equation

$$u[n] = w[n] \cdot (\sin(\Omega_0 n) + k), \qquad (1)$$

where $w[n]$ is the weight window with length $N_w$ samples, and also the total length of the pulse $u[n]$, for which holds that $N_w < T_p \cdot f_s$, $\Omega_0$ is the central normalized pulse frequency and $k > 1$ is a constant. The pulse $u[n]$ generated in this way has its energy concentrated into the band with its center on the frequency $\Omega_0$ and the frequency band width that can be controlled by the choice of the window $w[n]$.

In the described embodiment of the invention, the transmitting section $\underline{V}$ provides irradiation of the monitored organ and contains $N$ transmitters $V_1$ to $V_N$, where each block irradiates the tissue by radiation with a specific wavelength. The receivers $P_1$ to $P_M$ of the receiving demodulator $P$ then detect the radiation reflected from the tissue at various places of the organ and convert the detected energy to an electric signal. The control section $\mu P$ ensures the control of the whole measurement process, generation of the excitation pulses for irradiation, and processing of the measured data.

For simultaneous measurement at several wavelengths, the time multiplex is used. The individual transmitters $V_1$ to $V_N$ are sequentially activated by the common switcher $S_u$ for the time period $T_p$, which is denoted as an elementary measuring interval and during which the measurement at a single wavelength is performed. During one elementary measuring interval, the activated transmitting block is excited by the pulse $u[n]$ with the sampling frequency $f_s$ according to the equation (1) stated above.

The individual transmitters $V_1$ to $V_N$ subsequently convert the pulse $u[n]$ to the emitted radiation, which then reflects from or transmits through the monitored tissue and is detected by the receivers $P_1$ to $P_M$. The received signal is subsequently filtered using matched filtering, which suppresses the frequencies not present in the excitation signal $u[n]$. Appropriate selection of the $\Omega_0$ and $w[n]$ allows for suppression of interfering effects, such as power noise interference and 1/f noise.

The function of each of the transmitters $V_1$ to $V_N$ is illustrated by the diagram in Fig. 2. The transmitters $V_1$ to $V_N$ convert the excitation pulse $u[n]$ by means of the digital-to-analog converter $DAC$ to the control voltage $u(t)$ of the voltage/current converter $V/I$, the output of which provides the current excitation $i(t)$ for the total of $L$ light-emitting diodes $D_1$ to $D_L$ connected in series to irradiate the monitored tissue at the total of $L$ various places simultaneously.

The function of each of the receivers $P_1$ to $P_M$ is illustrated by the diagram in Fig. 3. Each receiver detects the radiation reflected from or transmitted through the tissue by means of its sensor $D$ and subsequently it converts the current of the sensor by means of the current/voltage converter $I/V$ to a voltage, which is filtered by the analog band-pass filter $PP$ in order to remove undesired components, such as low-frequency noises, e.g. 1/f noise. The analog band-pass filter $PP$ simultaneously serves as an anti-aliasing filter for the analog-to-digital converter $ADC$, which converts the received signal into a digital form.

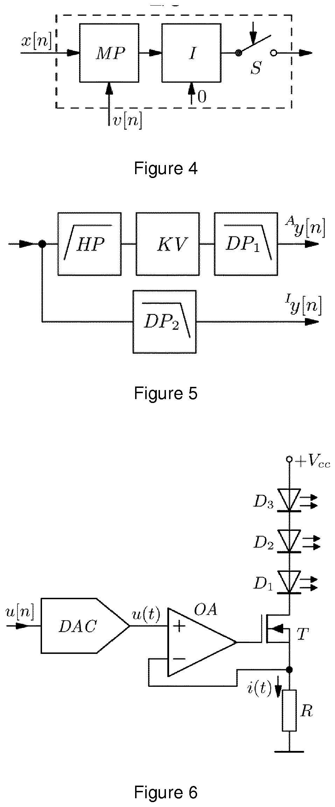

As mentioned above, the control section $\mu P$ has several functions. The reflected radiation is measured at several wavelengths using the time multiplex where the use of synchronized switching of the common switcher $S_u$ and the switchers $S_1$ to $S_M$ achieves synchronous irradiation of the organ and reception of reflected radiation, always within a single elementary measurement interval with the duration $T_p$. The matched filtering for the optimal detection of received pulses of radiation is implemented in the blocks of demodulators $DM_1$ to $DM_M$, the block diagram of which is shown in Fig. 4. In each of the demodulators $DM_1$ to $DM_M$ the digitized pulse $x[n]$ is multiplied by the pulse $v[n]$ by means of the multiplier $MP$. The pulse $v[n]$ is synthesized by filtering the pulse $u[n]$ with digital filter $H(z)$. The multiplication result is integrated over the entire elementary measurement interval and after its completion, the resulting value is passed over to the subsequent processing via the sampling switch $S$, which serves to capture the resulting sample after demodulation. Subsequently, the integrator is reset before the next integration. From the samples of signals thus obtained, which correspond to the reflected radiation at $N$ wavelengths, the blocks of the feature estimators $E_{i1}$ to $E_{iN}$ continuously estimate the features for classification of the state of blood circulation in the organ. Each of the feature estimators $E_{i1}$ to $E_{iN}$, see Fig. 5, processes the signal in two parallel branches. One branch estimates slow changes of the reflected radiation intensity by filtering the signal in the low-pass filter $DP_2$. The other branch estimates the amplitude of pulsations by means of the cascade connection of the high-pass filter $HP$, the squarer circuit $KV$, and the low-pass filter $DP_1$. The output signals of the first branch and the second branch respectively, of the estimator $E_{ij}$ will further be marked as $^I y_{ij}[n]$, and $^A y_{ij}[n]$ respectively. The state of blood supply to the measured organ is determined in the block of classifier $K$ based on the evaluation of the non-linear kernel of the classifier for the vectors of the selected input features and the monitoring of the time evolution of this metrics. The non-linear kernel of the classifier $K$ takes on the form of the function

$$ f(^I y_{11}[n], \dots, ^I y_{1N}[n], \dots, ^I y_{MN}[n], ^A y_{11}[n], \dots, ^A y_{1N}[n], \dots, ^A y_{MN}[n]), $$

where for example the multi-dimensional Gauss function may be used as the non-linear function $f()$.

An example of the preferred circuitry implementation of the transmitters $V_1$ to $V_N$ is shown in Fig. 6. The input of the transmitter is connected to the digital-to-analog converter $DAC$, the output of which is then connected to the inverting input of the operational amplifier $OA$, which together with the transistor $T$ and the resistor $R$ form a controlled current source for excitation of the light-emitting diodes $D_1$ to $D_L$ connected in series.

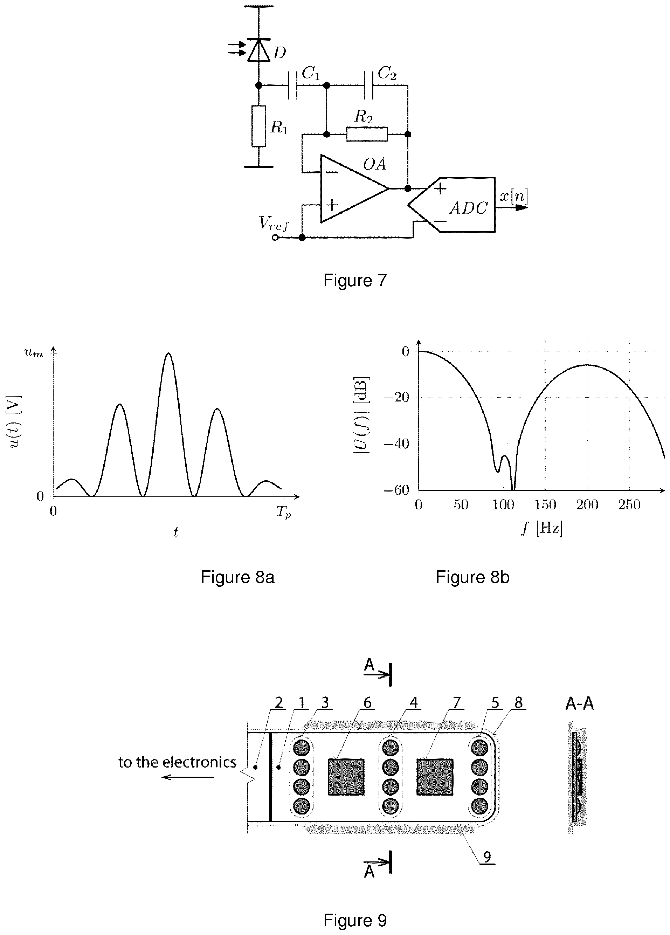

An example of the preferred circuitry implementation of the receivers $P_1$ to $P_M$ is shown in Fig. 7. The photosensitive sensor $D$ is connected to the resistor $R_1$, which together with the capacitors $C_1$ and $C_2$, resistor $R_2$, and the operational amplifier $OA$ forms a current-to-voltage converter with frequency transmission, which acts as a band-pass filter. Its output is against the reference voltage $V_{ref}$ connected to the input of the analog-to-digital converter $ADC$.

The mechanical design of the unit with sensors serves as a robust and flexible platform for the light emitters and sensors with the ability to conform to the shape of the monitored organ. It creates a stable homogeneous optical link between the optical sensors and the illuminated surface and ensures the waterproof separation of the electronics from the living tissue. In addition, it is designed in such a way that when the subject's monitoring is finished, it is possible to easily remove the complete set out from the body along with the drains without any additional surgical intervention.

The mechanical arrangement of the unit with sensors is illustrated in Fig. 9. The base is a printed circuit board 1 in the form of a flexible strip, which holds the individual parts and interconnecting elements 2 - that is a cable or a flexible printed circuit interconnecting the electrical elements on a printed circuit board inside the body with the electronics located outside the body. On the printed circuit board 1 the rows of the light-emitting diodes $D_1$ to $D_L$ are located, where each row includes one diode for each wavelength used. The number of light-emitting diodes $D_1$ to $D_L$ in series in each of the transmitters $V_1$ to $V_N$ corresponds to the number of rows of diodes in the mechanical arrangement, thus determining the size of the monitored volume of the tissue. Between these series of light-emitting diodes there are photosensitive sensors $\underline{D}$ of the receivers $P_1$ to $P_M$. The complete set is then encapsulated in the optically transparent package $\underline{8}$ up to the connector connecting this part to the control electronics. Moreover, on the sides of the optically transparent package $\underline{8}$ extensions $\underline{9}$ are created, providing increased stability of the unit with sensors and possible fixation to the monitored organ. The unit with sensors can be fixed by suturing these extensions to the organ in such a way that will not harm the monitored organ when removing the strip out from the body.

Industrial Applicability

The invention can be used for monitoring of changes of the function of internal organs using minimally invasive methods. For example, the invention is intended for monitoring perfusion of a transplanted renal graft in an early postoperative period. Its advantage is a robust detection of perfusion changes in the monitored organ. A simple structure of the unit with sensors with a flexible profile for conformation to the surface of the monitored organ inside a living body during surgery, the possibility of continuous monitoring of an organ in the postoperative period and the subsequent easy removal of the unit with sensors out of the body together with the drains without surgical intervention make this system an attractive candidate for inclusion to the indispensable equipment of the postoperative departments in hospitals.

**Claims**

1.  A system for monitoring the blood supply to a transplanted organ consists of a transmitting section ($\underline{V}$) with transmitters ($V_1$ to $V_N$) with light-emitting diodes ($D_1$ to $D_L$), which irradiate the monitored tissue by radiation at various wavelengths, of a receiving section ($\underline{P}$) with receivers ($P_1$ to $P_M$) with a photosensitive sensor ($\underline{D}$), which detects the radiation reflected from the monitored tissue, and of a control section ($\underline{\mu P}$), which generates excitation pulses ($u[n]$) for the transmitters ($V_1$ to $V_N$) and processes the signals from the receivers ($P_1$ to $P_M$), **characterized by the fact that** each of the transmitters ($V_1$ to $V_N$), which are $N$ in total, where $N$ corresponds to the number of wavelengths used for irradiation, comprises at its input a digital-to-analog converter ($\underline{DAC}$) connected to the input of a voltage/current converter ($\underline{V/I}$), the output of which is connected to at least one light-emitting diode $D_1$ to $D_L$ for the wavelength of the given transmitter ($V_1$ to $V_N$), and if multiple light-emitting diodes ($D_1$ to $D_L$) are used, they are connected in series between the output of the voltage/current converter ($\underline{V/I}$) and the power supply voltage ($+V_{cc}$), each of the receivers ($P_1$ to $P_M$) in the receiving section ($\underline{P}$), which are $M$ in total, where $M$ is given by the requirement for the volume of the monitored organ or its part, is formed by the photosensitive sensor ($\underline{D}$), the output of which is connected via a current/voltage converter ($\underline{I/V}$) and via an analog band-pass filter ($\underline{PP}$) to the input of an analog-to-digital converter ($\underline{ADC}$), while its output is the output of the given receiver, and each of the outputs of the receivers ($P_1$ to $PM$) is connected to the first input of its respective demodulator ($DM_1$ to $DM_M$) in the control section ($\underline{\mu P}$), the demodulators ($DM_1$ to $DM_M$) comprise at their inputs the multiplier ($\underline{MP}$) connected via the integrator ($\underline{I}$) with resetting and via a sampling switch ($\underline{S}$) to the input of their relevant switchers ($S_1$ to $S_M$), while each of them has $N$ outputs and each of these outputs is connected to one feature estimator ($E_{i1}$ to $E_{iN}$) for each wavelength, and each feature estimator ($E_{i1}$ to $E_{iN}$) is formed by two parallel branches, where one branch is formed at its input by a high-pass filter ($\underline{HP}$), the output of which is connected via a squarer circuit ($\underline{KV}$) to the input of the first low-pass filter ($DP_1$), and the output of the first low-pass filter ($DP_1$) is the output of the signal ($^Ay[n]$) corresponding to the amplitude of pulsations of the radiation reflected at the given wavelength, and the second branch is formed at its input by the second low-pass filter ($DP_2$) and its output is the output of the signal ($^Iy[n]$) corresponding to slow changes of the reflected radiation intensity, and the outputs of all feature estimators ($E_{i1}$ to $E_{iN}$) are connected to a classifier ($\underline{K}$), while the control section ($\underline{\mu P}$) further contains a digital filter ($\underline{H(z)}$) and its transfer function corresponds to the discretized transfer function of the analog band-pass filter ($\underline{PP}$), where the output of the digital filter ($\underline{H(z)}$) is connected to the second inputs of the demodulators ($DM_1$ to $DM_M$), and its input is connected to the output of the generator ($\underline{G}$), which is simultaneously connected to the input of the common switcher ($S_u$), which has the outputs connected to the inputs of the individual transmitters ($V_1$ to $V_N$) so they can be sequentially activated during the elementary measuring interval $T_p$ by the pulse $u[n]$ with the sampling frequency $f_s$ according to the equation

$$u[n] = w[n] \cdot (\sin(\Omega_0 n) + k),$$

where $w[n]$ is the weight window with length $N_w$ samples, $\Omega_0$ is the central normalized frequency of the pulse, and $k > 1$ is a constant, while for the total length of the pulse $u[n]$ holds that $T_p \cdot f_s > N_w$.

**2.** The system according to claim 1 **characterized by the fact that** the mechanical design of the unit with sensors of the system that is applied on the monitored organ is implemented in the form of a flexible strip that carries the light-emitting diodes ($D_1$ to $D_L$) and photosensitive sensors ($D$), and the light-emitting diodes are arranged into $L$ groups where each group includes one source for each wavelength, and the photosensitive sensors ($D$) are placed between the individual groups, while the whole unit is encapsulated in an optically transparent package (8).

**Patentansprüche**

**1.** System zur Überwachung der Blutversorgung des transplantierten Organs, bestehend aus einem Sendeteil ($V$) mit Sendern ($V_1$ bis $V_N$) mit Leuchtdioden ($D_1$ bis $D_L$) zur Bestrahlung des überwachten Gewebes durch Strahlung verschiedener Wellenlängen, mit Empfängerteil ($\overline{P}$) mit Empfängern ($P_1$ bis $P_M$) mit lichtempfindlichem Sensor ($D$) zum Erfassen der vom überwachten Gewebe reflektierenden Strahlung und mit Steuerteil ($\mu P$), zur Erzeugung von Anregungsimpulsen ($u[n]$) für Sender ($V_1$ bis $V_N$) und Signalverarbeitung von Empfängern ($P_1$ bis $P_M$), **gekennzeichnet dadurch, dass** jeder der Sender ($V_1$ bis $V_N$), deren Anzahl $N$ der Anzahl der verwendeten Wellenlängen für die Bestrahlung entspricht, am Eingang mit einem Digital-Analog-Umsetzer ($DAC$) gebildet ist, angeschlossen am Eingang des Umsetzers Spannung/Strom ($V/I$), auf dessen Ausgang für die Wellenlänge des gegebenen Senders ($V_1$ bis $V_N$) mindestens eine Leuchtdiode ($D_1$ bis $D_L$) angeschlossen ist, wobei im Falle von mehreren Leuchtdioden ($D_1$ bis $D_L$) sind diese in der Serie zwischen dem Ausgang des Umsetzers Spannung/Strom ($V/I$) und der Versorgungsspannung ($+V_{cc}$) angeschlossen, jeder der Empfänger ($P_1$ bis $P_M$) des Empfängerteils ($P$), deren Anzahl $M$ durch die Anforderung für das Volumen des überwachten Organs oder seines Teils gegeben ist, wird aus lichtempfindlichem Sensor ($D$) gebildet, dessen Ausgang über den Umsetzer Strom/Spannung ($I/V$) und über Analog-Bandpass ($PP$) am Eingang vom Analog-Digital-Umsetzer ($ADC$) angeschlossen ist, dessen Ausgang der Ausgang des gegebenen Empfängers ist, und jeder der Ausgänge der Empfänger ($P_1$ bis $P_M$) ist am ersten Eingang seines Demodulators ($DM_1$ bis $DM_M$) des Steuerteils ($\mu P$) angeschlossen, gebildet am Eingang aus einem Multiplikator ($MP$), verbunden über einen Integrator ($I$) und über einen Abtastschalter ($S$) mit dem Eingang seines Umschalters ($S_1$ bis $S_M$), von denen jeder N eigene Ausgänge hat und an jedem von ihnen ist für jede Wellenlänge ein Schätzer ($E_{i1}$ bis $E_{iN}$) der Symptome angeschlossen, von denen jeder durch zwei parallele Zweige gebildet ist, wo einer am Eingang aus Hochpass ($HP$) besteht, dessen Ausgang über einen Quadrierer ($KV$) am Eingang des ersten Tiefpasses ($DP_1$) angeschlossen ist, dessen Ausgang der Ausgang des Signals ($^Ay[n]$) ist, entsprechend der Pulsierungsamplitude der reflektierenden Strahlung an entsprechender Wellenlänge und der zweite Zweig besteht am Eingang aus zweitem Tiefpass ($DP_2$), dessen Ausgang der Ausgang des Signals ($^Iy[n]$) ist, entsprechend langsamen Änderungen der Intensität der reflektierenden Strahlung, und die Ausgänge aller Schätzer ($E_{i1}$ bis $E_{iN}$) der Symptome sind am Klassifizierer ($K$) angeschlossen, wobei der Steuerteil ($\mu P$) weiterhin ein digitales Filter ($H(z)$) umfasst, dessen Übertragungsfunktion der diskretisierten Filterübertragung vom Analog-Bandpass ($PP$) entspricht, wo der Ausgang des digitalen Filters ($H(z)$) mit den zweiten Ausgängen der Demodulatoren ($DM_1$ bis $DM_M$) verbunden ist und an seinem Eingang ist der Ausgang des Generators (G) angeschlossen, der zugleich mit dem Eingang des gemeinsamen Umschalters ($S_u$) verbunden ist, mit Ausgängen, angeschlossen an einzelnen Sendern ($V_1$ bis $V_N$) zum Zweck der schrittweisen Aktivierung für elementares Messintervall $T_p$ durch Puls $u[n]$ mit Abtastfrequenz $f_s$ nach Beziehung

$$u[n] = w[n] \cdot (\sin(\Omega_0 n) + k),$$

wo $w[n]$ das Längenwägungsfenster $N_w$ der Proben, $\Omega_0$ eine zentrale standardisierte Pulsfrequenz ist und $k > 1$ ist eine Konstante, wobei für die Gesamtlänge des Impulses $u[n]$ gilt, dass $T_p \cdot f_s > N_w$.

**2.** System nach Anspruch 1 **gekennzeichnet dadurch, dass** die mechanische Struktur des sensorischen Systemteils für das Anlegen an das überwachte Organ in Form eines flexiblen Streifens mit zwei Leuchtdioden ($D_1$ bis $D_L$) und lichtempfindlichen Sensoren ($D$) ist, wobei die Leuchtdioden ($D_1$ bis $D_L$) in $L$ Gruppen angeordnet ist, in jeder Gruppe eine Quelle von jeder Wellenlänge anwesend ist, und zwischen einzelnen Gruppen sind lichtempfindliche Sensoren ($D$) angeordnet, wobei dieser ganzer Satz in einer optisch transparenten Verpackung (8) eingekapselt ist.

**Revendications**

**1.** Le système de monitoring de l'approvisionnement en sang de l'organe transplanté créé par une partie d'émission ($V$) avec les des émetteurs (de $V_1$ à $V_N$) ayant les diodes lumineuses (de $D_1$ à $D_L$) pour la radiation du tissu surveillé en rayonnant par les différentes longueurs d'onde, partie réceptrice ($\underline{P}$) avec récepteurs (de $P_1$ à $P_M$) ayant un capteur translucide (**$\underline{D}$**) pour la détection de la radiation repoussée du tissu surveillé et par une partie dirigeante ($\underline{\mu P}$), pour la génération des impulsions d'excitation ($u[n]$) pour les émetteurs (de $V_1$ à $V_N$) et le traitement des signaux des récepteurs (de $P_1$ à $P_M$), **qui sont caractérisés par le fait que** chaque émetteur (de $V_1$ à $V_N$), dont le nombre $N$ ne répond pas au nombre des longueurs d'onde utilisées pour la radiation, il est créé à l'entrée par un convertisseur digital-analogique ($\underline{DAC}$) connecté à l'entrée du convertisseur tension/courant ($\underline{V/I}$), à l'entrée duquel est connecté pour la longueur d'onde de l'émetteur donné (de $V_1$ à $V_N$) au minimum une diode lumineuse (de $D_1$ à $P_L$), quand, s'il y a plusieurs diodes lumineuses, (de $D_1$ à $D_L$) celles-ci sont connectées en série entre la sortie du convertisseur tension/courant ($\underline{V/I}$) et la tension d'alimentation (+$V_{cc}$), chaque émetteur (de $P_1$ à $P_M$) de la partie réceptrice ($\underline{P}$), dont le nombre $M$ est donné par la revendication concernant la grandeur du volume de l'organe surveillé ou d'une sa partie, il est créé par un capteur translucide ($\underline{D}$), la sortie duquel est connectée à travers le convertisseur tension/courant ($\underline{I/V}$) et un filtre passe-bande analogique ($\underline{PP}$) sur l'entrée du convertisseur digital-analogique ($\underline{ADC}$), dont la sortie est sortie du récepteur donné et chaque sortie des récepteurs (de $P_1$ à $P_M$) est connecté avec la première entrée de son démodulataire (de $DM_1$ à $DM_M$) dirigeante les parties ($\underline{\mu P}$) créé sur l'entrée par un multiplicateur ($\underline{MP}$) interconnecté à travers l'intégrateur ($\underline{I}$) avec la remise à zéro et à travers l'interrupteur d'échantillonnage ($\underline{S}$) avec l'entrée de propre interrupteur (de $S_1$ à $S_M$), dont chacun d'eux a numéro $N$ de propres sorties et sur chacune d'elles est connecté pour chaque longueur d'onde un estimateur (de $E_{i1}$ à $E_{iN}$) des indices, dont chacun est créé par deux branches parallèles où l'une consiste en entrée du filtre passe-haut ($\underline{HP}$) dont la sortie est connectée à travers le quadrateur ($\underline{KV}$) avec l'entrée du premier filtre passe-bas ($DP_1$) dont la sortie est la sortie du signal ($^A y[n]$) correspondante à l'amplitude des impulsions de la radiation repoussée sur une longueur d'onde correspondante et l'autre branche est créée à l'entrée par un second filtre passe-bas ($DP_2$) dont la sortie est la sortie du signal ($^I y[n]$) correspondante aux modifications lentes de l'intensité de la radiation repoussé, et les sorties de tous les estimateurs (de $E_{i1}$ à $E_{iN}$) des indices sont branchés dans le classificateur ($\underline{K}$) quand la partie dirigeante ($\underline{\mu P}$) contient aussi un filtre digital ($H(\underline{z})$) la fonction de transmission duquel répond à la transmission discrétisée du filtre du filtre passe-bande analogique ($\underline{PP}$) où la sortie du filtre digital ($H(\underline{z})$) est interconnecté avec les autres entrées des démodulateurs (de $DM_1$ à $\underline{DMM)}$ et sur son entrée est connectée la sortie du générateur ($\underline{G}$) qui est en même temps interconnecté avec l'entrée de l'interrupteur commun ($S_u$) ayant les sorties connectées avec les différents émetteurs (de $V_1$ à $V_N$) dans le but de leur activation graduelle jusqu'à l'intervalle élémentaire de mesurage $T_p$ par impulsion $u[n]$ avec la fréquence d'échantillon $f_s$ selon le rapport

$$u[n] = w[n] \cdot (\sin(\Omega_0 n) + k),$$

où $w[n]$ est la fenêtre pondérale de la longueur $N_w$ des échantillons, $\Omega_0$ est la fréquence centrale standardisée d'impulsion et $k > 1$ est la constante, quand pour la longueur totale d'impulsion $u[n]$ est valable que $T_p \cdot f_s > N_w$.

2. Le système selon la revendication 1 **caractérisé par le fait que** la construction mécanique de la partie sensorielle du système pour la pose sur l'organe surveillé et en forme d'une bande élastique munie des diodes lumineuses (de $D_1$ à $D_L$) et des capteurs translucides ($\underline{D}$) quand les diodes lumineuses (de $D_1$ à $D_L$) sont rangées dans les groupes $\overline{L}$ où dans chaque groupe est présente une source de chaque longueur d'onde et parmi les différents groupes sont installés les capteurs translucides ($\underline{D}$) quand cet ensemble entier est gainé dans une enveloppe optiquement transparente (8).

Figure 1

Figure 2

Figure 3

Figure 4

Figure 5

Figure 6

Figure 7

Figure 8a

Figure 8b

to the electronics

Figure 9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2015208923 A1
- WO 2015070162 A2
- US 2009156918 A1
- US 4653498 A
- US 4167331 A
- US 4883353 A
- US 5792052 A
- EP 2442709 A
- EP 0329196 A
- EP 1237467 A
- EP 2022394 A
- US 8150487 B
- US 8718737 B
- US 9351673 B
- US 5482036 A
- US 5490505 A
- US 5431159 A
- US 7062307 B
- EP 1709902 A
- US 5349952 A

**Non-patent literature cited in the description**

- Implantable pulse oximetry on subcutaneous tissue. **THEODOR ; MICHAEL et al.** Engineering in Medicine and Biology Society (EMBC), 2014 36th Annual International Conference of the IEEE. IEEE, 2014
- Pulse oximetry of body cavities and organs. **KYRIACOU, P. A. ; M. HICKEY ; J. P. PHILLIPS.** Engineering in Medicine and Biology Society (EMBC), 2013 35th Annual International Conference of the IEEE. IEEE, 2013
- **SHAH ; NITIN et al.** Performance of three new-generation pulse oximeters during motion and low perfusion in volunteers. *Journal of clinical anesthesia,* 2012, vol. 24.5, 385-391
- **BARKER ; STEVEN J.** Motion-resistant'' pulse oximetry: a comparison of new and old models. *Anesthesia & Analgesia,* 2002, vol. 95.4, 967-972
- **REICHELT ; STEPHAN et al.** Development of an implantable pulse oximeter. *IEEE transactions on Biomedical Engineering,* 2008, vol. 55.2, 581-588
- Engineering in Medicine and Biology Society (EMBC). 2014 36th Annual International Conference of the IEEE. IEEE, 2014